(19) Europäisches Patentamt
European Patent Office
Office européen des brevets

(11) EP 0 330 756 B1

(12) **EUROPÄISCHE PATENTSCHRIFT**

(45) Veröffentlichungstag und Bekanntmachung des Hinweises auf die Patenterteilung: **27.03.1996 Patentblatt 1996/13**

(51) Int. Cl.[6]: **A61K 31/55**, C07D 495/04 // (C07D495/04, 333:00, 243:00)

(21) Anmeldenummer: **88121220.3**

(22) Anmeldetag: **19.12.1988**

(54) **Thienotricyclen zur Behandlung von Erkrankungen der Bronchien**

Thienotricyclics for the treatment of bronchial diseases

Composés thiénotricycliques pour le traitement des maladies bronchiques

(84) Benannte Vertragsstaaten:
**AT BE CH DE ES FR GB GR IT LI LU NL SE**

(30) Priorität: **22.12.1987 CH 4991/87**
**11.07.1988 CH 2648/88**

(43) Veröffentlichungstag der Anmeldung:
**06.09.1989 Patentblatt 1989/36**

(73) Patentinhaber: **Byk Gulden Lomberg Chemische Fabrik GmbH**
**D-78403 Konstanz (DE)**

(72) Erfinder:
- **Grundler, Gerhard**
**D-7750 Konstanz (DE)**
- **Ulrich, Wolf-Rüdiger**
**D-7750 Konstanz (DE)**
- **Figala, Volker**
**D-7753 Allensbach 4 (DE)**
- **Klemm, Kurt**
**D-7753 Allensbach (DE)**
- **Schudt, Christian**
**D-7750 Konstanz (DE)**
- **Boer, Rainer**
**D-7750 Konstanz (DE)**
- **Eltze, Manfrid**
**D-7750 Konstanz (DE)**
- **Riedel, Richard**
**D-7967 Bad Waldsee-Reute (DE)**
- **Beume, Rolf**
**D-7750 Konstanz 18 (DE)**
- **Galvan, Martin**
**D-7750 Konstanz 18 (DE)**
- **Kılian, Ulrich**
**D-7752 Reichenau 2 (DE)**
- **Rainer, Georg**
**D-7750 Konstanz (DE)**

(56) Entgegenhaltungen:
**EP-A- 0 039 519** **EP-A- 0 214 528**
**WO-A-86/06278**

- **EUR. J. PHARMACOL., Band 112, Nr. 2, 7. Juni 1985, Seiten 211-224; Elsevier Sc. Publ. B.V.; M. ELTZE et al.: "Pharmacological evidence for selective inhibition of gastric acid secretion by telenzepine, a new antimuscarinic drug"**
- **GUT, Band 28, Nr. 7, Juli 1987, Seiten 888-895; W. LONGDONG et al.: "Telenzepine is at least 25 times more potent than pirenzepine - a dose response and comparative secretory study in man"**
- **EUR. J. PHARMACOL., Band 133, Nr. 1, 6. Januar 1987, Seiten 21-27; Elsevier Sc. Publ. B.V.; J.W. BLOOM et al.: "A muscarinic receptorwith high affinity for pirenzepine mediates vagally induced bronchoconstriction"**
- **PHARMACOLOGY, Band 37, Suppl. 1, 1988, Seiten 32-39, Workshop on M1-selective muscarinic antagonists in peptic ulcer therapy, Constance, DE, October 2-3, 1987; C. SCHMUDT et al.: "Does the prolonged occupancy of M1 receptors by telenzepine protect them against the action of vagally released acetylcholine?"**
- **PHARMACOLOGY, Band 37, Suppl. 1, 1988, Seiten 40-47, Workshop on M1-selective muscarinic antagonists in peptic ulcer therapy, Constance, DE, October 2-3, 1987; M. ELTZE: "Assessment of selectivity of muscarinic antagonists for M1 and M2 receptors in rabbit isolated vas deferens"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Rank Xerox (UK) Business Services
2.9.16/3.4

- **INT. J. CLIN. PHARMACOL. THER. TOXICOL., Band 24, Nr. 12, Dezember 1986, Seiten 655-657; K. SERTL et al.: "Acute effects of pirenzepin on bronchospasm"**
- **J. PHARMACOL. EXP. THER., Band 247, Nr. 1, Oktober 1988, Seiten 136-142; T. SCOTT et al.: "Antagonists and agonists interactions with the muscarinic receptor of the rat large airways"**
- **POSTGRAD. MED. J., Band 63, Suppl. 1, 1987, Seiten 75-78, Anticholinergic Therapy - The state of the art; proceedings of a symposium held in Stratford-upon-Avon, September 8-9, 1986; T.W. HIGENBOTTAM: "Anticholinergics and cough"**
- **AM. REV. RESPIR. DIS., Band 138, Nr. 4, 1988, Seiten 765-767; N.J. GROSS et al.: "A short tour around the muscarinic receptor"**

Bemerkungen:

Verbunden mit 89900602.7/0386145 (europäische Anmeldenummer/Veröffentlichungsnummer) durch Entscheidung vom 28.11.90.

**Beschreibung**

Die Erfindung betrifft die neue Verwendung eines Dihydrothienobenzodiazepinons zur Herstellung von Arzneimitteln für die Behandlung von Bronchialerkrankungen, die enantiomer reinen Formen dieses Dihydrothienobenzodiazepinons, Verfahren zu ihrer Herstellung sowie ihre Verwendung.

Aus dem europäischen Patent 0039519 sind Dihydrothienobenzodiazepinone und ihre Anwendung bei der Behandlung von Krankheiten des Magens oder Darms bekannt. Weiterhin ist bekannt, daß Dibenzodiazepinone bzw. Pyridobenzodiazepinone in zwei zueinander spiegelbildlichen chiralen Konformationen vorliegen können, wobei sich die Konformerengemische in bestimmten Fällen - je nach Substitution - in die optischen Antipoden aufspalten lassen (DE-OS 35 31 682).

Es wurde nun gefunden, daß ein im europäischen Patent 0039519 beschriebenes Dihydrothienobenzodiazepinon, nämlich die Verbindung 4,9-Dihydro-3-methyl-4-[(4-methyl-1-piperazinyl)acetyl]-10H-thieno[3,4-b][1,5]benzodiazepin-10-on (INN: (INN: Telenzepin), unerwartet ausgeprägte bronchospasmolytische Eigenschaften besitzt. Es wurde weiterhin ein Verfahren gefunden, durch das sich geeignet substituierte Dihydrothienobenzodiazepinone, insbesondere Telenzepin, glatt und in hohen Ausbeuten in ihre optischen Antipoden aufspalten lassen.

Ein Gegenstand der Erfindung ist daher die Verwendung des 4,9-Dihydro-3-methyl-4-[(4-methyl-1-piperazinyl)acetyl]-10H-thieno[3,4-b][1,5]benzodiazepin-10-ons und seiner pharmakologisch verträglichen Salze zur Herstellung von Arzneimitteln zur Behandlung und Prophylaxe von Erkrankungen der Bronchien.

Weiterer Gegenstand der Erfindung ist die Verwendung des (+)-Enantiomeren von Telenzepin, also des (+)-4,9-Dihydro-3-methyl-4-[(4-methyl-1-piperazinyl)ace tyl]-10H-thieno[3,4-b][1,5]benzodiazepin-10-ons und seiner pharmakologisch verträglichen Salze zur Herstellung von Arzneimitteln zur Behandlung und Prophylaxe von Erkrankungen der Bronchien.

Weiterer Gegenstand der Erfindung sind Arzneimittel, die die Verbindung (+)-4,9-Dihydro-3-methyl-4-[(4-methyl-1-piperazinyl)-acetyl]-10H-thieno-[3,4-b][1,5]benzodiazepin-10-on und/oder ihre pharmakologisch verträglichen Salze enthalten.

Als Salze kommen in diesem Zusammenhang bevorzugt pharmakologisch verträgliche Salze mit in der Galenik üblicherweise verwendeten anorganischen und organischen Säuren in Betracht. Beispielsweise seien die Salze mit Fumarsäure (Fumarate), Weinsäure (Tartrate) oder Maleinsäure (Maleate) genannt. Als bevorzugtes Säureadditionssalz sei das Dihydrochlorid erwähnt.

Die Arzneimittel werden nach an sich bekannten Verfahren hergestellt, wobei die Verbindungen als solche oder gegebenenfalls in Kombination mit geeigneten pharmazeutischen Hilfs- oder Trägerstoffen in Form von Tabletten, Dragees, Kapseln, Suppositorien, Pflastern (z.B. als TTS), Emulsionen, Suspensionen, Aerosolen oder Lösungen eingesetzt werden. Enthalten die neuen pharmazeutischen Zubereitungen neben dem Wirkstoff pharmazeutische Trägerstoffe, so beträgt der Wirkstoffgehalt dieser Mischungen 0,5 bis 95, vorzugsweise 15 bis 75 Gewichtsprozent der Gesamtmischung.

Welche Hilfs- bzw. Trägerstoffe für die gewünschten Arzneimittelformulierungen geeignet sind, ist dem Fachmann aufgrund seines Fachwissens geläufig. Neben Lösemitteln, Gelbildnern, Suppositoriengrundlagen, Tabletten-Hilfsstoffen und anderen Wirkstoffträgern können beispielsweise Antioxidantien, Dispergiermittel, Lösungsvermittler, Farbstoffe oder insbesondere Permeationspromotoren und Komplexbildner (z.B. Cyclodextrine) verwendet werden.

Die Arzneimittel werden in jeder geeigneten Formulierung angewandt unter der Voraussetzung, daß die Ausbildung bzw. Aufrechterhaltung von ausreichenden Wirkstoffspiegeln gewährleistet ist. Das kann beispielsweise durch orale oder parenterale Gabe in geeigneten Dosen erreicht werden. Üblicherweise liegt die pharmazeutische Zubereitung des Wirkstoffs in Form von Einheitsdosen vor, die auf die gewünschte Verabreichung abgestimmt sind. Eine Einheitsdosis kann zum Beispiel eine Tablette, ein Dragée, eine Kapsel, ein Suppositorium oder eine gemessene Volumenmenge eines Pulvers, eines Granulates, einer Lösung, einer Emulsion oder eine Suspension sein.

Unter "Einheitsdosis" im Sinne der vorliegenden Erfindung wird eine physikalisch bestimmte Einheit, die eine individuelle Menge des aktiven Bestandteils in Kombination mit einem pharmazeutischen Trägerstoff enthält, verstanden, deren Wirkstoffgehalt einem Bruchteil oder Vielfachen einer therapeutischen Einzeldosis entspricht. Eine Einzeldosis enthält vorzugsweise die Menge Wirkstoff, die bei einer Applikation verabreicht wird und die gewöhnlich einer ganzen, einer halben, einer drittel oder einer viertel Tagesdosis entspricht. Wenn für eine einzelne therapeutische Verabreichung nur ein Bruchteil, wie die Hälfte oder ein Viertel, der Einheitsdosis benötigt wird, ist die Einheitsdosis vorteilhafterweise teilbar, z.B. in Form einer Tablette mit Bruchkerbe.

Die pharmazeutischen Zubereitungen gemäß der Erfindung können, wenn sie in Einheitsdosen vorliegen und für die Applikation z.B. am Menschen bestimmt sind, etwa 0,1 bis 10 mg, vorteilhafterweise 0,5 bis 5 mg und insbesondere 1 bis 4 mg Wirkstoff enthalten.

Die therapeutische Verabreichung der pharmazeutischen Zubereitung kann 1 bis 4 mal am Tage zu festgelegten oder variierenden Zeitpunkten erfolgen, z.B. jeweils vor den Mahlzeiten und/oder am Abend. Es kann jedoch erforderlich sein, von den genannten Dosierungen abzuweichen, und zwar in Abhängigkeit von der Art, dem Körpergewicht und dem Alter des zu behandelnden Individuums, der Art und Schwere der Erkrankung, der Art der Zubereitung und der

Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Bei akuten Fällen wird zu Beginn der Behandlung eine höhere Dosis verabreicht. Nach Eintreten der gewünschten Wirkung wird auf eine niedrigere Dosis zurückgegangen.

Die Festlegung der jeweils erforderlichen optimalen Dosierung und Applikationsart der Wirkstoff kann durch jeden Fachmann aufgrund seines Fachwissens erfolgen.

Die pharmazeutischen Zubereitungen bestehen in der Regel aus dem Wirkstoff und nichttoxischen, pharmazeutisch verträglichen Arzneimittelträgern, die als Zumischung oder Verdünnungsmittel in fester, halbfester oder flüssiger Form oder als Umhüllungsmittel, beispielsweise in Form einer Kapsel, eines Tablettenüberzugs, eines Beutels oder eines anderen Behältnisses, für den therapeutisch aktiven Bestandteil in Anwendung kommen. Ein Trägerstoff kann z.B. als Vermittler für die Arzneimittelaufnahme durch den Körper, als Formulierungshilfsmittel, als Süßungsmittel, als Geschmackskorrigens, als Farbstoff oder als Konservierungsmittel dienen.

Zur oralen Anwendung können z.B. Tabletten, Dragées, harte und weiche Kapseln, z.B. aus Gelatine, dispergierbare Pulver, Granulate, wässerige und ölige Suspensionen, Emulsionen, Lösungen oder Sirupe kommen.

Tabletten können inerte Verdünnungsmittel, z.B. Calciumcarbonat, Calciumphosphat, Natriumphosphat oder Lactose; Granulierungs- und Verteilungsmittel, z.B. Maisstärke oder Alginate; Bindemittel, z.B. Stärke, Gelatine oder Akaziengummi; und Gleitmittel, z.B. Aluminium- oder Magnesiumstearat, Talkum oder Silikonöl, enthalten. Sie können zusätzlich mit einem Überzug versehen sein, der auch so beschaffen sein kann, daß er eine verzögerte Auflösung und Resorption des Arzneimittels im Gastrointestinaltrakt bewirkt, so daß z.B. eine bessere Verträglichkeit, Protrahierung oder eine Retardierung erreicht wird. Gelatinekapseln können den Arzneistoff vermischt mit einem festen, z.B. Calciumcarbonat oder Kaolin, oder einem öligen, z.B. Oliven-, Erdnuß- oder Paraffinöl, Verdünnungsmittel enthalten.

Wäßrige Suspensionen, die gegebenenfalls kurzfristig zubereitet werden, können Suspendiermittel, z.B. Natriumcarboxymethylcellulose, Methylcellulose, Hydroxypropylcellulose, Natriumalginat, Polyvinylpyrrolidon, Traganthgummi oder Akaziengummi; Dispergier- und Benetzungsmittel, z.B. Polyoxyethylenstearat, Heptadecaethylenoxycetanol, Polyoxyethylensorbitolmonooleat, Polyoxyethylensorbitanmonooleat oder Lecithin; Konservierungsmittel, z.B. Methyl- oder Propylhydroxybenzoate; Geschmacksmittel; Süßungsmittel, z.B. Saccharose, Lactose, Natriumcyclamat, Dextrose, Invertzuckersirup, enthalten.

Ölige Suspensionen können z.B. Erdnuß-, Oliven-, Sesam-, Kokos- oder Paraffinöl und Verdickungsmittel, wie z.B. Bienenwachs, Hartparaffin oder Cetylalkohol, enthalten; ferner Süßungsmittel, Geschmacksmittel und Antioxidantien.

In Wasser dispergierbare Pulver und Granulate können die Arzneistoffe in Mischungen mit Dispergier-, Benetzungs- und Suspendiermitteln, z.B. den oben genannten, sowie mit Süssungsmitteln, Geschmacksmitteln und Farbstoffen enthalten.

Emulsionen können z.B. Oliven-, Erdnuß- oder Paraffinöl neben Emulgiermitteln, wie z.B. Akaziengummi, Traganthgummi, Phosphatiden, Sorbitanmonooleat, Polyoxyethylensorbitanmonooleat, und Süßungs- und Geschmacksmitteln enthalten.

Zur rektalen Anwendung der Arzneistoffe werden Suppositorien verwendet, die mit Hilfe von bei Rektaltemperatur schmelzenden Bindemitteln, beispielsweise Kakaobutter oder Polyethylenglykol, hergestellt werden.

Zur parenteralen Anwendung der Arzneistoffe dienen steril injizierbare, gegebenenfalls kurzfristig herzustellende, wäßrige Suspensionen, isotonische Salzlösungen oder sonstige Lösungen, die Dispergier- oder Benetzungsmittel und/oder pharmakologisch verträgliche Verdünnungsmittel, z.B. Propylen- oder Butylenglycol, enthalten.

Die orale Anwendung der Arzneistoffe ist bevorzugt.

Bevorzugt für die Verwendung als Bronchospasmolytikum ist auch die inhalative Applikation der Wirkstoffe. Diese werden entweder direkt als Pulver oder durch Vernebeln von Lösungen oder Suspensionen, die die Wirkstoffe enthalten, verabreicht. Die Vernebelung kann dabei auf herkömmliche Weise, beispielsweise durch Preßluftvernebler oder Ultraschallvernebler, erfolgen. Besonders vorteilhaft ist die Verabreichung aus Sprühdosen, insbesondere solcher mit einem herkömmlichen Dosierventil (Dosier-Aerosole). Mittels Dosier-Aerosolen ist es möglich, pro Sprühstoß eine definierte Menge an Wirkstoff bereitzustellen. Von besonderem Vorteil sind hier sogenannte Synchron-Inhalatoren, womit die Wirkstoffgabe synchron zur Einatmung geschehen kann. Geeignete Synchron-Inhalationsvorrichtungen sind z.B. in der DE-PS 1945257, DE-PS 1917911 und DE-OS 2055734 offenbart.

Für Inhalationszwecke kommen die Wirkstoffe vorzugsweise in mikronisierter Form zum Einsatz, wobei Teilchengrößen von weniger als 10 µm vorteilhaft sind. Zur Applikation aus Sprühdosen werden die Wirkstoffe in üblichen Treibmitteln dispergiert, vorzugsweise unter Zuhilfenahme eines Dispergiermittels. Als Treibmittel kommen insbesondere Gemische von Trichlorfluormethan (Frigen®11) und Dichlordifluormethan (Frigen®12) in Frage, wobei Trichlorfluormethan ganz oder teilweise durch 1,1,2-Trichlortrifluorethan (Frigen®113) ersetzt werden kann. Als Dispergiermittel kommen insbesondere die für diese Zwecke gebräuchlichen Sorbitanester (Spane® der Firma Atlas GmbH) und Lecithin in Frage. Das Dispergiermittel wird in der gekühlt vorgelegten schwerer flüchtigen Treibmittelkomponente gelöst. In die Lösung wird der mikronisierte Wirkstoff bzw. werden die mikronisierten Wirkstoffe eingerührt. Die Dispersion wird in Sprühbüchsen eingefüllt. Nach dem Vercrimpen wird die leichter flüchtige Treibmittelkomponente aufgedrückt.

Der oder die Wirkstoffe können gegebenenfalls mit einem oder mehreren der angegebenen Träger- oder Zusatzstoffe auch in mikroverkapselter Form formuliert werden.

Als Erkrankungen der Bronchien seien beispielsweise chronische abstruktive Atemwegserkrankungen verschiedener Genese (z.B. Bronchitis, Asthma bronchiale) beim Mensch oder Tier genannt, die aufgrund der hervorragenden bronchospasmolytischen Eigenschaften mit Telenzepin bzw. (+)-Telenzepin behandelt werden können. Gegenstand der Erfindung ist somit auch ein Verfahren zur Behandlung von Säugern, insbesondere Menschen, die an den Bronchien erkrankt sind. Das Verfahren ist dadurch gekennzeichnet, daß man dem an den Bronchien erkrankten Individuum eine therapeutisch wirksame und pharmakologisch verträgliche Menge an Telenzepin bzw. (+)-Telenzepin verabreicht.

Telenzepin ist aus dem europäischen Patent 0039519 bekannt. Das (+)-Enantiomere von Telenzepin wird nach einem neuen Verfahren hergestellt, das ebenfalls Gegenstand der Erfindung ist. Dieses Verfahren, für das die in der DE-OS 3531682 offenbarte Enantiomerentrennung über die diastereomeren Salze keinerlei Anregung bieten konnte, ist dadurch gekennzeichnet, daß man Telenzepin (= Verbindung der Formel I) als solches oder in seiner deprotonierten Form

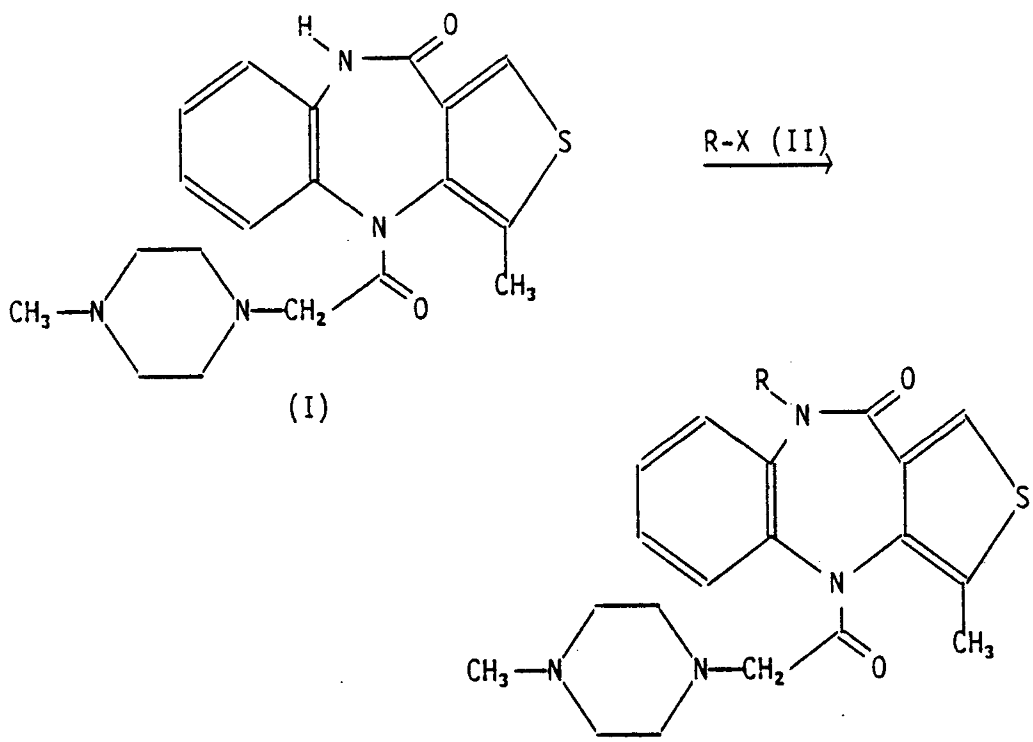


(I)

(III)

mit einer konfigurativ einheitlichen chiralen Verbindung der Formel II, worin R einen konfigurativ einheitlichen, chiralen Rest und X eine Abgangsgruppe darstellt, umsetzt, das erhaltene Diastereomerengemisch III trennt und aus den optisch reinen Diastereomeren die konformativ einheitlichen Verbindungen I freisetzt.

Als Verbindungen der Formel II kommen prinzipiell alle chiralen, konfigurativ einheitlichen Verbindungen infrage, die mit der Verbindung I oder ihrem Anion unter Abspaltung der Abgangsgruppe X zu reagieren in der Lage sind und deren Rest R nach der Diastereomerentrennung glatt und ohne unerwünschte Nebenreaktionen wieder abgespalten werden kann.

Als Abgangsgruppen X kommen insbesondere alle nucleophil ablösbaren Atome oder Gruppen, wie beispielsweise Halogenatome (J, Br oder insbesondere Cl) oder durch Veresterung (z.B. mit Sulfonsäuren) aktivierte Hydroxylgruppen ($-O-SO_2-CH_3$, $-O-SO_2-CF_3$ oder $-O-SO_2-C_6H_4-p-CH_3$) in Frage.

Als Reste R kommen alle konfigurativ einheitlichen Reste infrage, die sich von natürlich vorkommenden oder synthetisch zugänglichen chiralen Verbindungen ableiten lassen und die solvolytisch unter milden, bevorzugt leicht sauren Bedin gungen aus den Verbindungen III abgespalten werden können. Als Reste R seien insbesondere genannt

- Glycosylreste, die sich von Glycopyranosen, Glycofuranosen oder Oligosacchariden ableiten und die gewünschtenfalls mit in der Kohlenhydratchemie üblichen Schutzgruppen teilweise oder vollständig geschützt sind, oder
- chirale, über das Sauerstoffatom verknüpfte Terpenalkoholreste, oder
- andere chirale, über das Sauerstoffatom verknüpfte Alkoholreste,

die jeweils an dem als Verknüpfungsglied fungierenden Sauerstoffatom eine Carbonylgruppe oder insbesondere eine Methylengruppe tragen.

Bevorzugte Reste R sind Reste der Formel IV

$$R1\text{-}O\text{-}CH_2\text{-} \qquad (IV)$$

worin R1 gemeinsam mit dem Sauerstoffatom, woran es gebunden ist, einen Glycosylrest, einen chiralen Terpenalkoholrest, oder einen sonstigen chiralen Alkoholrest darstellt.

Als Glycosylreste R1-O- seien beispielsweise die Reste genannt, die sich von natürlich vorkommenden Mono- oder Disacchariden, wie Arabinose, Fructose, Galactose, Glucose, Lactose, Mannose, Ribose, Xylose, Maltose, Sorbose oder N-Acetyl-D-glucosamin herleiten.

Als chirale Terpenalkoholreste R1-O- seien insbesondere solche Reste genannt, die sich von einem natürlich vorkommenden oder synthetisch leicht zugänglichen Terpenalkohol herleiten. Als beispielhafte Terpenalkohole seien hierbei genannt: Isopulegol, Neomenthol, Isomenthol, Menthol, Carveol, Dihydrocarveol, Terpinen-4-ol, Mirtenol, Citronellol, Isoborneol, Borneol, Fenchol und insbesondere Isopinocampheol.

Als sonstige chirale Alkoholreste R1-O- seien beispielsweise die Reste genannt, die sich von folgenden Alkoholen herleiten: Mandelsäureester, Cinchonidin, Cinchonin, Ephedrin, Serinmethylester, Sitosterol, 3-Hydroxy-2-methyl-propionsäuremethylester und Milchsäureethylester.

Ein besonderes bevorzugter Rest R ist der Isopinocampheyloxymethylrest.

Die Umsetzung der Verbindung I mit der Verbindung II erfolgt auf eine dem Fachmann vertraute Weise. Zur Erhöhung der Nucleophilie der Verbindung I ist es zweckmäßig, diese zu deprotonieren. Als Deprotonierungsmittel können die üblicherweise für analoge Reaktionen in der organischen Chemie verwendeten Basen eingesetzt werden, so zum Beispiel Hydroxide, wie Natriumhydroxid oder Kaliumhydroxid, oder Carbonate, wie Kaliumcarbonat, oder Alkoholate, wie Natriummethylat oder Natriumethylat, oder Amine, wie Pyridin, Triethylamin oder Ethyldiisopropylamin, oder metallorganische Verbindungen, wie tert.-Butyllithium, oder vorzugsweise Hydride, wie Natriumhydrid.

Die Deprotonierung der Verbindung I und anschließende Umsetzung mit Verbindung II wird in inerten - je nach Deprotonierungsmittel - protischen oder aprotischen Lösungsmitteln durchgeführt. Als solche eignen sich beispielsweise Methanol, Isopropanol, Dimethylsulfoxid, Aceton, Acetonitril, Dioxan und vorzugsweise Dimethylformamid.

Die Deprotonierung der Verbindung I wird - in Abhängigkeit von der Reaktivität des Deprotonierungsmittels - vorzugsweise bei Temperaturen zwischen -30°C und +100°C, insbesondere bei Temperaturen zwischen 0°C und 50°C durchgeführt. Die anschließende Umsetzung der deprotonierten Verbindung I mit Verbindung II erfolgt bei Temperaturen die - zur Vermeidung unerwünschter Nebenreaktionen - 50°C nicht überschreiten sollten und vorzugsweise zwischen -20°C und +20°C liegen.

Die Trennung des nach der Umsetzung von I mit II erhaltenen Diastereomerengemisches erfolgt in an sich bekannter Weise, beispielsweise durch Chromatografie an geeigneten Säulen oder vorzugsweise durch fraktionierte Kristallisation. Falls die Trennung durch Kristallisation erfolgen soll, ist es zweckmäßig, die erhaltenen Distereomeren in geeignete, leicht kristallisierende Salze zu überführen. Als solche eignen sich beispielsweise das Oxalat, das Tosilat, das Fumarat und insbesondere das Maleat.

Die Freisetzung der Verbindungen III aus ihren Salzen nach erfolgter Diastereomerentrennung wird mit Hilfe geeigneter, nicht zu starker Basen vorgenommen. Hierfür eignen sich beispielsweise Hydrogencarbonate, wie Natriumhydrogencarbonat.

Die Freisetzung der konformativ einheitlichen Verbindungen I aus den optisch reinen Diastereomeren erfolgt durch Solvolyse unter schonenden, leicht sauren Bedingungen. Als für die Solvolyse geeignetes Reagens hat sich beispielsweise wasserfreie Ameisensäure mit einem Zusatz an Chlorwasserstoff erwiesen.

Die Verbindungen der Formel II sind bekannt bzw. sie sind auf eine für den Fachmann vertraute Weise aus bekannten Verbindungen auf analoge Weise zugänglich. So können beispielsweise die Verbindungen II, in denen R die Bedeutung der Formel IV hat und X ein Chloratom darstellt, durch Chlormethylierung entsprechender Alkohole [z.B. in Analogie zu R.C. Ronald et al., J. Org. Chem. 45 (1980) 2224] hergestellt werden.

Die nach Freisetzung aus den optisch reinen Diastereomeren erhaltenen konformativ einheitlichen Verbindungen I, die das linear polarisierte Licht der Wellenlänge 589 nm in (+)- bzw. (-)-Richtung drehen, also das (+)-4,9-Dihydro-3-methyl-4-[(4-methyl-1-piperazinyl)-acetyl]-10H-thieno-[3,4-b][1,5]benzodiazepin-10-on und das (-)-4,9-Dihydro-3-methyl-4-[(4-methyl-1-piperazinyl)-acetyl]-10H-thieno-[3,4-b][1,5]benzodiazepin-10-on und ihre Salze sind neue Verbindungen mit überraschenden pharmakologischen Eigenschaften.

Weiterer Gegenstand der Erfindung ist daher das (+)-4,9-Dihydro-3-methyl-4-[(4-methyl-1-piperazinyl)-acetyl]-10H-thieno-[3,4-b][1,5]benzodiazepin-10-on und seine Salze.

Ebenso ist das (-)-4,9-Dihydro-3-methyl-4-[(4-methyl-1-piperazinyl)-acetyl]-10H-thieno-[3,4-b][1,5]benzodiazepin-10-on und seine Salze Gegenstand der Erfindung.

Als Salze kommen in diesem Zusammenhang bevorzugt pharmakologisch verträgliche Salze mit in der Galenik üblicherweise verwendeten anorganischen und organischen Säuren in Betracht. Als bevorzugtes Säureadditionssalz sei das Dihydrochlorid genannt.

Weiterer Gegenstand der Erfindung sind die im erfindungsgemäßen Verfahren als Zwischenprodukte auftretenden Verbindungen der Formel III, worin R die oben angegebene Bedeutung hat, und ihre Salze.

Die folgende Beispiele erläutern die Erfindung näher, ohne sie einzuschränken.

## Beispiele

**1.** 4,9-Dihydro-9-[(+)-isopinocampheyloxymethyl]-3-methyl-4-[(4-methyl-1-piperazinyl)-acetyl]-10H-thieno[3,4-b][1,5]benzodiazepin-10-on

109,6 g 4,9-Dihydro-3-methyl-4-[(4-methyl-1-piperazinyl)-acetyl]-10H-thieno-[3,4-b][1,5]benzodiazepin-10-on werden unter Stickstoffatmosphäre in 2,2 l wasserfreiem Dimethylformamid suspendiert und auf 40° erwärmt. Innerhalb von 2,5 Stunden werden 14,2 g Natriumhydrid (80 %ige Suspension in Paraffinöl) unter Rühren zugegeben. Anschließend wird die Mischung 16 Stunden bei Raumtemperatur gerührt. Danach wird auf +5°C abgekühlt, und es werden 41,6 g (+)-Isopinocampheyl-chlormethylether während 30 Minuten zugetropft. Die Mischung wird noch weitere 30 Minuten bei 5°C gerührt und anschließend tropfenweise mit 150 ml Eisessig versetzt. Die Lösung wird am Rotationsverdampfer eingeengt. Der Rückstand wird in 300 ml Wasser gelöst. Die Lösung wird zunächst mit 2 N HCl auf pH 1-2 gestellt. Nach 5 Minuten wird solange festes Natriumbicarbonat zugegeben, bis ein pH-Wert von 9 erreicht wird. Nach Extraktion mit 3 x 200 ml Essigester, Trocknen über Natriumsulfat und Einengen erhält man 97,0 g Rohprodukt als zähes Öl. Zur Reinigung wird an Kieselgel chromatographiert. (Fließmittel: Toluol/Dioxan/Methanol= 70 : 15 : 15). 76,1 g (69 %) der Titelverbindung werden als farbloser, amorpher Feststoff isoliert. ($[\alpha]_D^{22}$ = +25,2°); c=1, Dichlormethan/Methanol = 1 : 1).

**2.** 4,9-Dihydro-9-[(-)-isopinocampheyloxymethyl]-3-methyl-4-[(4-methyl-1-piperazinyl)-acetyl]-10H-thieno[3,4-b][1,5]benzodiazepin-10-on

Nach der für Beispiel 1 angegebenen Vorschrift werden, ausgehend von 10,0 g 4,9-Dihydro-3-methyl-4-[(4-methyl-1-piperazinyl)-acetyl]-10H-thieno[3,4-b][1,5] benzodiazepin-10-on und 4,1 g (-)-Isopinocampheyl-chlormethylether, 6,3 g (58 %) der Titelverbindung als farbloser, amorpher Feststoff isoliert ($[\alpha]_D^{22}$ = -23,1°; c=1, Dichlormethan/Methanol = 1 : 1).

**3.** (+)-4,9-Dihydro-9-[(+)-isopinocampheyloxymethyl]-3-methyl-4-[(4-methyl-1-piperazinyl)-acetyl]-10H-thieno[3,4-b][1,5]benzodiazepin-10-on-maleat

76,0 g 4,9-Dihydro-9-[(+)-isopinocampheyloxymethyl]-3-methyl-4-[(4-methyl-1-piperazinyl)-acetyl]-10H-thieno[3,4-b][1,5]benzodiazepin-10-on und 16,4 g Maleinsäure werden unter Erwärmen (auf 70°C) in 1,5 l Ethanol gelöst. Aus dieser Lösung kristallisieren nach 16 Stunden bei Raumtemperatur und 1 Stunde bei +10°C 19,2 g (20 %) der Titelverbindung aus. Schmp.: 205°C; ($[\alpha]_D^{22}$ = +98,2°; c=1, Dichlormethan/Methanol = 1 : 1).

**4.** (-)-4,9-Dihydro-9-[(-)-isopinocampheyloxymethyl]-3-methyl-4-[(4-methyl-1-piperazinyl)-acetyl]-10H-thieno[3,4-b][1,5]benzodiazepin-10-on-maleat

5,8 g 4,9-Dihydro-9-[(-)-isopinocampheyloxymethyl]-3-methyl-4-[(4-methyl-1-piperazinyl)-acetyl]-10H-thieno[3,4-b][1,5]benzodiazepin-10-on und 1,25 g Maleinsäure werden unter Erwärmen (70°C) in 140 ml Ethanol gelöst. Aus dieser Lösung kristallisieren nach 16 Stunden bei Raumtemperatur und 1 Stunde bei 0°C 2,1 g eines Diastereomerengemischs mit ($[\alpha]_D^{22}$ = -77°; c=1, Dichlormethan/Methanol = 1 : 1) aus. 1,8 g dieses Gemischs werden in 30 ml Ethanol umkristallisiert. 850 mg (14 %) der Titelverbindung werden als farbloses Kristallisat vom Schmp.: 207°C isoliert ($[\alpha]_D^{22}$ = -96,4°; c=1, Dichlormethan/Methanol = 1 : 1).

**5.** (+)-4,9-Dihydro-9-[(+)-isopinocampheyloxymethyl]-3-methyl-4-[(4-methyl-1-piperazinyl)-acetyl]-10H-thieno[3,4-b][1,5]benzodiazepin-10-on

10,0 g (+)-4,9-Dihydro-9-[(+)-isopinocampheyloxymethyl]-3-methyl-4-[(4-methyl-1-piperazinyl)-acetyl]-10H-thieno[3,4-b][1,5]benzodiazepin-10-on-maleat werden in 100 ml Wasser suspendiert und mit 1,4 g Natriumbicarbonat versetzt. Die Lösung wird mit 3 x 100 ml Essigester extrahiert; die organischen Extrakte werden über Natriumsulfat getrocknet und anschließend eingeengt. 8,1 g (99 %) der Titelverbindung werden als farbloser, amorpher Feststoff isoliert. ($[\alpha]_D^{22}$ = +96,1°; c=1, Dichlormethan/Methanol = 1 : 1).

**6.** (-)-4,9-Dihydro-9-[(-)-isopinocampheyloxymethyl]-3-methyl-4-[(4-methyl-1-piperazinyl)-acetyl]-10H-thieno[3,4-b][1,5]benzodiazepin-10-on

Nach der für Beispiel 5 beschriebenen Vorschrift werden 700 mg (-)-4,9-Dihydro-9-[(-)-isopinocampheyloxymethyl]-3-methyl-4-[(4-methyl-1-piperazinyl)-acetyl]-10H-thieno[3,4-b][1,5]benzodiazepin-10-on-maleat und 200 mg Natriumbicarbonat umgesetzt. 550 mg (96 %) der Titelverbindung werden als farbloser, amorpher Feststoff isoliert. ($[\alpha]_D^{22}$ = -96,6°; c=1, Dichlormethan/Methanol= 1 : 1).

**7.** (+)-4,9-Dihydro-3-methyl-4-[(4-methyl-1-piperazinyl)-acetyl]-10H-thieno-[3,4-b][1,5]benzodiazepin-10-on

6,6 g (+)-4,9-Dihydro-9-[(+)-isopinocampheyloxymethyl]-3-methyl-4-[(4-methyl-1-piperazinyl)-acetyl]-10H-thieno[3,4-b][1,5]benzodiazepin-10-on werden bei Raumtemperatur in 66 ml mit Chlorwasserstoff versetzter Ameisensäure (trockenes Chlorwasserstoffgas wird bei Raumtemperatur 5 Minuten in 100 ml Ameisensäure eingeleitet) gelöst. Nach 5 Minuten wird das Lösungsmittel am Rotationsverdampfer abgezogen und der Rückstand in 100 ml Wasser aufgenommen. Mit 2 N HCl wird die Lösung auf pH 1-2 gestellt und dann mit 3 x 50 ml Dichormethan extrahiert. Anschließend wird die wässrige Phase mit Natriumbicarbonat auf pH 9 gestellt und mit 3 x 100 ml Dichlormethan extrahiert. Nach Trocknen über Natriumsulfat werden die organischen Extrakte eingeengt. Der Rückstand wird durch Chromatographie an Kieselgel (Laufmittel: Toluol/Dioxan/Methanol = 70 : 15 : 30) gereinigt. 3,5 g (67 %) der Titelverbindung werden als farbloser, amorpher Feststoff isoliert. ($[\alpha]_D^{22}$ = +41,2°; c=1, Dichlormethan/Methanol = 1 : 1; $[\alpha]_D^{22}$ = +32,4°; c=1 Chloroform; das Dihydrochlorid (2 HCl x 1,1 $H_2O$) hat einen Schmp. von 223-229°C (Zers.); $[\alpha]_D^{22}$ = +26,4°; c=1, Wasser).

**8.** (-)-4,9-Dihydro-3-methyl-4-[(4-methyl-1-piperazinyl)-acetyl]-10H-thieno-[3,4-b][1,5]benzodiazepin-10-on

400 mg (-)-4,9-Dihydro-9-[(-)-isopinocampheyloxymethyl]-3-methyl-4-[(4-methyl-1-piperazinyl)-acetyl]-10H-thieno[3,4-b][1,5]benzodiazepin-10-on werden, wie für Beispiel 7 beschrieben, in 4 ml Ameisensäure/HCl umgesetzt und gereinigt. 250 mg (90 %) der Titelverbindung werden als farbloser, amorpher Feststoff isoliert. ($[\alpha]_D^{22}$ = -41,0°; c=1, Dichlormethan/Methanol = 1 : 1; $[\alpha]_D^{22}$ = -32,5°; c=1, Chloroform; das Dihydrochlorid (2 HCl x $H_2O$) hat einen Schmp. von 223-229°C (Zers.); $[\alpha]_D^{22}$ = -26,3°; c=1, Wasser).

Gewerbliche Anwendbarkeit

Die Verbindungen 4,9-Dihydro-3-methyl-4-[(4-methyl-1-piperazinyl)acetyl]-10H-thieno[3,4-b][1,5]benzodiazepin-10-on und (+)-4,9-Dihydro-3-methyl-4-[(4-methyl-1-piperazinyl)acetyl]-10H-thieno[3,4-b][1,5]benzodiazepin-10-on und ihre pharmakologisch verträglichen Salze besitzen neue, bisher unbekannte pharmakologische Eigenschaften, die sie gewerblich verwertbar machen. So erweisen sich diese Verbindungen als überaus wertvoll für die Behandlung von Bronchialerkrankungen und sind daher für den Einsatz in der Human- und Veterinärmedizin hervorragend geeignet.

Die ausgezeichneten bronchialtherapeutischen Eigenschaften der beiden Verbindungen kommen insbesondere in einer einzigartigen bronchospasmolytischen Aktivität zum Ausdruck. Diese bronchospasmolytische Aktivität kann schon bei der Verabreichung von sehr geringen Dosen beobachtet werden. Besonders überraschend ist in diesem Zusammenhang die hohe Spezifität, die nach i.v.-Injektion kontinuierlich einsetzende und nicht überschießende Wirkung sowie vor allem die sehr lang anhaltende bronchospasmolytische Wirkung, die die Substanzen auf hervorragende Weise für die Therapie asthmatischer Erkrankungen geeignet erscheinen läßt, wobei aufgrund der unerwarteten und überragend langen Wirkungsdauer die Substanzen insbesondere für eine Therapie nächtlicher Asthmaanfälle in Frage kommen.

In ihrer ausgezeichneten Wirksamkeit, die einhergeht mit dem Fehlen wesentlicher Nebenwirkungen und einem nur geringen Einfluß auf das cardiovaskuläre System, erweisen sich die Verbindungen (±)-Telenzepin und (+)-Telenzepin den aus dem Stand der Technik bekannten Bronchospasmolytica deutlich überlegen. Hervorzuheben ist, daß (±)-Telenzepin und (+)-Telenzepin als bisher einzige Verbindungen dieses Wirktyps nach intravenöser, intratrachealer (i.t.) und auch nach oraler Gabe gut wirken.

Weiterhin erweist sich die Verbindung (+)-4,9-Dihydro-3-methyl-4-[(4-methyl-1-piperazinyl)-acetyl]-10H-thieno[3,4-b][1,5]benzodiazepin-10-on als stark wirksames Antimuscarinikum, das aufgrund seiner überlegenen antiulcerogenen und magensäuresekretionshemmenden Eigenschaften für den Einsatz in der Human- und Veterinärmedizin hervorragend geeignet ist. Überraschenderweise zeigt das (+)-4,9-Dihydro-3-methyl-4-[(4-methyl-1-piperazinyl)acetyl]-10H-thieno[3,4-b][1,5]benzodiazepin-10-on wesentlich ausgeprägtere muscarin-antagonistische Eigenschaften als das (-)-4,9-Dihydro-3-methyl-4-[(4-methyl-1-piperazinyl)-acetyl]-10H-thieno[3,4-b][1,5]benzodiazepin-10-on, wie es in Rezeptor-Affinitätsstudien nachgewiesen werden konnte.

Die Verbindung (+)-4,9-Dihydro-3-methyl-4-[(4-methyl-1-piperazinyl)-acetyl]-10H-thieno[3,4-b][1,5]benzodiazepin-10-on hemmt deutlich die Magensäuresekretion von Warmblütern und weist darüberhinaus eine ausgezeichnete Magen- und Darmschutzwirkung bei Warmblütern auf. Diese Magen- und Darmschutzwirkung wird bereits bei der Verabreichung

von Dosen beobachtet, die unterhalb der säuresekretionshemmenden Dosen liegen. Darüberhinaus zeichnet sich die Verbindung (+)-4,9-Dihydro-3-methyl-4-[(4-methyl-1-piperazinyl)-acetyl]-10H-thieno-[3,4-b][1,5]benzodiazepin-10-on durch das Fehlen wesentlicher Nebenwirkungen und eine große therapeutische Breite aus.

Unter "Magen- und Darmschutz" wird in diesem Zusammenhang die Verhütung und Behandlung gastrointestinaler Krankheiten, insbesondere gastrointestinaler entzündlicher Krankheiten und Läsionen (wie z.B. Ulcus ventriculi, Ulcus duodeni, Gastritis, hyperazider oder medikamentös bedingter Reizmagen) verstanden, die beispielsweise durch Mikroorganismen, Bakterientoxine, Medikamente (z.B. bestimmte Antiphlogistika und Antirheumatika), Chemikalien (z.B. Ethanol), Magensäure oder Streßsituationen verursacht werden können.

In ihren ausgezeichneten Eigenschaften erweist sich die Verbindung (+)-4,9-Dihydro-3-methyl-4-[(4-methyl-1-piperazinyl)-acetyl]-10H-thieno[3,4-b][1,5]benzodiazepin-10-on überraschenderweise dem aus dem Stand der Technik (EP 0039519) bekannten Konformerengemisch, also dem (±)-4,9-Dihydro-3-methyl-4-[(4-methyl-1-piperazinyl)-acetyl]-10H-thieno[3,4-b][1,5]benzodiazepin-10-on, überlegen. Aufgrund dieser Eigenschaften ist das (+)-Konformere und seine pharmakologisch verträglichen Salze auch für den Einsatz in der Human- und Veterinärmedizin zur Behandlung und/oder Prophylaxe von Krankheiten des Magens und Darms und solcher Krankheiten, die auf einer überhöhten Magensäuresekretion beruhen, hervorragend geeignet.

Ein weiterer Gegenstand der Erfindung sind daher die Verbindungen (+)-4,9-Dihydro-3-methyl-4-[(4-methyl-1-piperazinyl)-acetyl]-10H-thieno[3,4-b][1,5]benzodiazepin-10-on zur Anwendung bei der Behandlung und/oder Prophylaxe von Krankheiten des Magens und Darms und solcher Krankheiten, die auf einer überhöhten Magensäuresekretion beruhen.

Ebenso umfaßt die Erfindung die Verwendung der Verbindung (+)-4,9-Dihydro-3-methyl-4-[(4-methyl-1-piperazinyl)-acetyl]-10H-thieno[3,4-b][1,5]benzodiazepin-10-on zur Herstellung von Arzneimitteln, die zur Behandlung und/oder Prophylaxe von Krankheiten des Magens und Darms und solcher Krankheiten, die auf einer überhöhten Magensäuresekretion beruhen, eingesetzt werden.

Soll die Verbindung (+)-4,9-Dihydro-3-methyl-4-[(4-methyl-1-piperazinyl)acetyl]-10H-thieno[3,4-b][1,5]benzodiazepin-10-on und/oder ihre Salze zur Behandlung und/oder Prophylaxe von Krankheiten des Magens und Darms und solcher Krankheiten, die auf einer überhöhten Magensäuresekretion beruhen, eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxyd, Magnesiumaluminat; andere, die Säuresekretion hemmende Pharmaka, wie beispielsweise $H_2$-Blocker (z.B. Cimetidin, Ranitidin); Tranquilizer, wie Benzodiazepine, beispielsweise Diazepam; Spasmolytika, wie z.B. Bietamiverin, Camylofin; Anticholinergica, wie z.B. Oxyphencyclimin, Phencarbamid; Lokalanaesthetika, wie z.B. Tetracain, Procain; gegebenenfalls auch Fermente, Vitamine oder Aminosäuren enthalten. Die Verbindung (+)-4,9-Dihydro-3-methyl-4-[(4-methyl-1-piperazinyl)acetyl]-10H-thieno[3,4-b][1,5]benzodiazepin-10-on kann darüberhinaus auch zur Verhütung gastrointestinaler Krankheiten in Kombination mit Wirkstoffen wie Antiphlogistika oder Antirheumatika eingesetzt werden, um deren ulcerogene Potenz zu antagonisieren und so Nebenwirkungen zu verringern. Weiterhin kann die Verbindung (+)-4,9-Dihydro-3-methyl-4-[(4-methyl-1-piperazinyl)acetyl]-10H-thieno[3,4-b][1,5]benzodiazepin-10-on in Kombination mit Wirkstoffen zur Behandlung von Krankheitszuständen, bei denen cholinerge Mechanismen wesentlich beteiligt sind (wie z.B. Asthma), verabfolgt werden.

Pharmakologie

Die überragende Wirkung von Telenzepin kann am Modell des Acetylcholin-induzierten Bronchospasmus am narkotisierten, spontanatmenden Meerschweinchen (i.v.-Injektion und i.t.-Instillation) sowie am wachen Meerschweinchen (p.o.-Applikation) nachgeApplikation) nachgewiesen werden:

Einfluß von i.v.-verabreichtem Telenzepin auf Acetylcholin-induzierte Bronchospasmen an narkotisierten, spontanatmenden Meerschweinchen.

Versuchsanordnung:

Methode zur simultanan Registrierung von pharmakodynamischen bzw. toxischen Effekten auf innere sensible Rezeptoren und die Atmung von Meerschweinchen (U. Kilian, E. Müller, E. Ch. Dittmann und J. Hamacher, Arzneim.-Forsch. 28, 1699-1708, 1978).

Meerschweinchen:

Pirbright White, Interfauna (Tuttlingen), 350-450 g, männlich.

Narkose:

Ethylurethan 1,2 g/kg i.p. als 12,5 %ige wäßrige Lösung.

Registrierung:

Pneumotachogramm: Maximale Strömungsgeschwindigkeiten der Atemluft während der Exspiration ($Vmax_e$, "peak flow"), Atemzugvolumen (TV).

Dosierung:

| µmol/kg i.v., gelöst in 0,9 %iger NaCl-Lösung | | | | | | | | |
|---|---|---|---|---|---|---|---|---|
| (±)-Telenzepin | 0,03 | 0,05 | 0,1 | | 1 | 5 | | |
| (+)-Telenzepin | 0,03 | 0,05 | 0,1 | 0,5 | 1 | | | |
| (-)-Telenzepin | | | 0,1 | | 1 | 5 | 10 | 30 |

Acetylcholin als Spasmogen 0,2 - 0,3 µmol/kg i.v., gelöst in 0,9 %iger NaCl-Lösung.

Versuchsablauf:

-10 min Kontrollspasmus, 0 min Substanzapplikation, +2, +10, +20, +30, +60 min Versuchsspasmen.

Auswertung:

Erfassen der Wirkung von Telenzepin auf die Meßparameter (Mittelwert und Standardfehler des Mittelwertes, n=10) und daraus Berechnung der bronchospasmolytischen Aktivität als prozentuale Hemmung der Acetylcholin-bedingten Abnahmen von $Vmax_e$ und TV mittels Zeitwirkungskurven. Rechnerische Ermittlung einer Dosiswirkungskurve der durchschnittlichen bronchospasmolytischen Aktivität während der Beobachtungszeit (0-60 min) aus den Flächen unter den Zeitwirkungskurven und Ablesen von 1h-$ED_{50}$-Werten. Ermittlung einer Dosiswirkungskurve der bronchospasmolytischen Aktivität zum Zeitpunkt 60 min und Ablesen von $ED_{50}$-Werten.

Ergebnisse:

(±)-Telenzepin und (+)-Telenzepin hemmen den Acetylcholin-bedingten Bronchospasmus dosis- und zeitabhängig in einem sehr niedrigen Dosisbereich von 0,03 - 1 µmol/kg i.v.; die Wirkung setzt trotz i.v.-Gabe bemerkenswert langsam ein und erreicht zwischen 30 und 10 min ein plateauartiges Maximum, welches über den Beobachtungszeitraum von 1 h anhält. Die Wirksamkeit von (-)-Telenzepin beginnt erst deutlich später zwischen 0,1 und 1 µmol/kg i.v. und erreicht zwischen 10 und 30 µmol/kg i.v. ein Maximum. Der Zeitwirkungsverlauf ist dem der beiden anderen Telenzepinformen vergleichbar.

Die Tabellen 1 und 2 beschreiben die Wirksamkeit von Telenzepin mittels Dosiswirkungskurven der durchschnittlichen Aktivität über den gesamten Beobachtungszeitraum von 0 - 60 min bzw. zum Zeitpunkt 60 min p.a.. Die daraus abgeleiteten $ED_{50}$-Werte sind in den Tabellen 3 und 4 wiedergegeben.

Tabelle 1

| Durchschnittliche bronchospasmolytische Aktivität von (±)-, (+)- und (-)-Telenzepin während der **gesamten** Beobachtungszeit (0-60 min), ausgegeben als prozentuale Steigerung der maximalen Strömungsgeschwindigkeiten der Atemluft während der Exspiration ($Vmax_e$) und des Atemzugvolumens (TV) im Acetylcholin-induzierten Versuchsspasmus im Vergleich zum Kontrollspasmus, $\bar{x}$ ± SEM, N = 10. | | | | | | | | | |
|---|---|---|---|---|---|---|---|---|---|
| | **μmol/kg i.v.** | **0,03** | **0,05** | **0,1** | **0,5** | **1** | **5** | **10** | **30** |
| $Vmax_e$ | (±)-Telenzepin | 7±3 | 22±3 | 61±8 | | 81±4 | 75±3 | | |
| | (+)-Telenzepin | 13±4 | 21±4 | 57±5 | 87±3 | 82±3 | | | |
| | (-)-Telenzepin | | | 2±1 | | 17±3 | 65±4 | 79±2 | 89±3 |
| TV | (±)-Telenzepin | 7±3 | 26±4 | 68±9 | | 84±4 | 86±2 | | |
| | (+)-Telenzepin | 17±4 | 25±5 | 62±5 | 92±3 | 85±3 | | | |
| | (-)-Telenzepin | | | 0±2 | | 20±5 | 74±4 | 87±2 | 98±3 |

Tabelle 2

| Bronchospasmolytische Aktivität von (±)-, (+)- und (-)-Telenzepin zum **Zeitpunkt 60 min p.a.**, ausgegeben als prozentuale Steigerung der maximalen Strömungsgeschwindigkeiten der Atemluft während der Exspiration ($Vmax_e$) und des Atemzugvolumens (TV) im Acetylcholin-induzierten Versuchsspasmus im Vergleich zum Kontrollspasmus, $\bar{x}$ + SEM, N = 10. | | | | | | | | | |
|---|---|---|---|---|---|---|---|---|---|
| | **μmol/kg i.v.** | **0,03** | **0,05** | **0,1** | **0,5** | **1** | **5** | **10** | **30** |
| $Vmax_e$ | (±)-Telenzepin | 11±4 | 25±3 | 70±9 | | 82±5 | 80±4 | | |
| | (+)-Telenzepin | 19±6 | 35±7 | 61±6 | 95±4 | 88±4 | | | |
| | (-)-Telenzepin | | | 1±1 | | 18±3 | 77±5 | 77±3 | 88±4 |
| TV | (±)-Telenzepin | 12±6 | 32±5 | 78±11 | | 85±5 | 91±3 | | |
| | (+)-Telenzepin | 23±7 | 39±7 | 64±7 | 100±4 | 88±4 | | | |
| | (-)-Telenzepin | | | -3±3 | | 22±5 | 88±6 | 89±3 | 94±3 |

Tabelle 3

| $ED_{50}$-Werte der **durchschnittlichen** bronchospasmolytischen Aktivität von (±)-, (+)- und (-)-Telenzepin | | | |
|---|---|---|---|
| | **$ED_{50}$ μmol/kg i.v.** | | |
| | **(±)-Telenzepin** | **(+)-Telenzepin** | **(-)-Telenzepin** |
| $Vmax_e$ | 0,08 | 0,1 | 3,1 |
| TV | 0,07 | 0,08 | 2,5 |

Tabelle 4

| $ED_{50}$-Werte bronchospasmolytischer Aktivität von (±)-, (+)- und (-)-Telenzepin **zum Zeitpunkt 60 min p.a.** | | | |
|---|---|---|---|
| | **$ED_{50}$ μmol/kg i.v.** | | |
| | **(±)-Telenzepin** | **(+)-Telenzepin** | **(-)-Telenzepin** |
| $Vmax_e$ | 0,08 | 0,07 | 3,2 |
| TV | 0,07 | 0,07 | 2,0 |

Einfluß von (±)-Telenzepin intratracheal (i.t.) auf Acetylcholin-bedingte Änderungen von Lungenfunktionsparametern des narkotisierten Meerschweinchens

Versuchsanordnung:

Kleintier-Ganzkörper-Plethysmographie an narkotisierten Meerschweinchen (Methode nach U.Heinrich und A.Wilhelm, in bga-Schriften 5/84, p 255-266, MMW-Verlag, 1984).

Meerschweinchen:

Pirbright White, Interfauna (Tuttlingen), 290-350 g, männlich.

Narkose:

Ethylurethan 1,2 g/kg i.p. als 12,5 %ige wäßrige Lösung.

Registrierung:

Plethysmographisch bei via naturalis geschobenem Trachealkatheter Strömungsgeschwindigkeit der Atemluft und Atemzugvolumen, über via naturalis geschobenen Ösophaguskatheter Messung des intrapleuralen Druckes.

Dosierung:

0,001, 0,01, 0,1 und 1 µmol/kg (±)-Telenzepin i.t., gelöst in 0,1 ml/kg 0,9 %ige NaCl + 0,1 % Tween 80, Acetylcholin als Spasmogen inhalativ durch Ultraschallvernebelung einer 0,18 %igen Ach/0,9 %igen NaCl-Lösung verabreicht.

Versuchsablauf:

-10 min Kontrollspasma, 0 min Substanzgabe, +5 und +30 min Versuchsspasmen.

Auswertung:

Rechnerische Ableitung von Conductance (=Leitfähigkeit der Atemwege für Luft, entspricht dem Kehrwert des Atemwegswiderstandes) und von Compliance (Lungendehnfähigkeit) aus den registrierten Parametern nach der Methode von Amdur and Mead (Amer.J.Physiol., 192, 364-368, 1958) vor und im Maximum des Spasmus und Bildung der Differenzen, Berechnung der prozentualen Hemmung des Spasmus nach Substanzgabe im Vergleich zur Kontrolle, Berechnung der Dosiswirkungskurve und der $ED_{50}$-Werte dieser spasmolytischen Aktivität.

Ergebnisse:

(±)-Telenzepin hemmt die Acetylcholin induzierte Abnahme von Conductance und Compliance zeit- und dosisabhängig in dem breiten, jedoch außerordentlich niedrigen Dosisbereich von 0,001 - 1 µmol/kg i.t.; die Wirksamkeit ist 30 min p.a. stärker ausgeprägt als 5 min p.a.

Die Tabelle 5 beschreibt die Wirksamkeit von (±)-Telenzepin 5 und 30 min p.a. mittels Dosiswirkungskurven. Die daraus abgeleiteten $ED_{50}$-Werte sind in Tabelle 6 zusammengefaßt.

Tabelle 5

Bronchospasmolytische Wirksamkeit von (±)-Telenzepin 5 und 30 min p.a., ausgedrückt als prozentuale Zunahme von Conductance und Compliance im Acetylcholin induzierten Versuchsspasmus im Vergleich zur Kontrolle ($\bar{x}$ ± SEM, N = 10).

| **µmol/kg i.t.** | | **0,001** | **0,01** | **0,1** | **1** |
|---|---|---|---|---|---|
| 5 min | Conductance | 12±3 | 62±10 | 46±10 | 65±9 |
| | Compliance | 5±1 | 50±9 | 34±10 | 57±9 |
| 30 min | Conductance | 20±5 | 51±9 | 54±7 | 84±2 |
| | Compliance | 13±3 | 49±9 | 51±9 | 89±4 |

Tabelle 6

| $ED_{50}$-Werte der bronchospasmolytischen Wirksamkeit von (±)-Telenzepin nach i.t. Instillation. | | |
|---|---|---|
| | **$ED_{50}$ µmol/kg i.t.** | |
| | **5 min p.a.** | **30 min p.a.** |
| Conductance | 0,05 | 0,03 |
| Compliance | 0,3 | 0,03 |

Einfluß von p.o.-verabreichtem (±)-Telenzepin auf Acetylcholin-induzierte Asthmaanfälle des wachen Meerscheinchens.

Versuchsanordnung:

Wie beschrieben bei R. Beume, U. Kilian, N. Kolassa, K. Mussler, Atemw.-Lungenkrkh., 11, 342-345, 1985 ("Die Prüfung bronchospasmolytischer Substanzen an wachen Meerschweinchen").

Meerschweinchen:

Pirbright White, Interfauna (Tuttlingen), 250-450 g, männlich, 10 Tiere pro Dosis und Versuch.

Registrierung und Auswertung:

Thorakographische Messung der Atemtätigkeit, Messung der Latenzzeit vom Beginn der Acetylcholin-Vernebelung bis zum Beginn des Asthmaanfalles; Feststellung der Zahl der Tiere, bei denen 30 min p.a. die Latenzzeit des Versuchs-Anfalles im Bezug zum Kontroll-Anfall wenigstens verdreifacht ist; daraus Abschätzung der ca. $ED_{50}$.

Dosierung:

0,1, 0,3, 1, 3, 10 und 30 µmol/kg (±)-Telenzepin p.o. (in 4 % Methocel in 0,9 % NaCl verabreicht); Acetylcholin 0,09 % in 0,9 % NaCl per inhalationem (Ultraschall-vernebelt).

Ergebnisse:

Die Zahl der Tiere, bei denen es wenigstens zu einer Verdreifachung der Latenzzeit des Acetylcholin-induzierten Versuchs-Anfalls im Vergleich zum Kontroll-Anfall in Abhängigkeit von der p.o.-applizierten (±)-Telenzepin-Dosis kommt, ist wie folgt dargestellt:

| **µmol/kg p.o.** | **Tierzahl** | |
|---|---|---|
| | **eingesetzt** | **Latenzzeit verdreifacht (=geschützt)** |
| 0,1 | 10 | 3 |
| 0,3 | 10 | 5 |
| 1 | 10 | 6 |
| 3 | 10 | 7 |
| 10 | 10 | 9 |
| 30 | 10 | 10 |

Daraus ergibt sich eine ca. $ED_{50}$ von 0,7 (0,3-1,7 95 % - Int.) µmol/kg p.o.

Rezeptor-Affinitätsstudien

Die Affinität von (±)-4,9-Dihydro-3-methyl-4-[(4-methyl-1-piperazinyl)-acetyl]10H-thieno[3,4-b][1,5]benzodiazepin-10-on (Telenzepin, in der folgenden Tabelle 7 Verbindung 2) und seinen Konformeren [(+)-Enatiomeres = Verbindung 1, (-)-Enantiomeres = Verbindung 3] zu muscarinischen $M_1$(ganglionärer Typ)-und $M_2$(cardialer Typ)-Rezeptoren wurde nach folgenden Methoden untersucht:

Methode 1: Elektrisch gereiztes Vas deferens des Kaninchens im Organbad (Eltze, M., 1988, Submitted for publication in Europ. J. Pharmacol.)

Registriert wurden isometrische Kontraktionen (HSE-Kraftaufnehmer, K-30; Kipp und Zonen, BD-9 Schreiber) isolierter Samenleiter-Abschnitte von Kaninchen (männliche, weiße Neuseeländer, 2,5 - 3,5 kg) im Organbad (10 ml, 31°C, Begasung mit Carbogen, Vorspannung der Organe 0,75 g) unter elektrischer Stimulation (HSE-Reizgerät; hakenförmige Pt-Elektrode 1 cm unter der Wasseroberfläche des Bades; 30 V, 0,5 ms, 0,05 Hz). Nach einer Äquilibrierungszeit von 30 Minuten wurden nach der von van Rossum (1963) beschriebenen Technik kumulative Dosiswirkungskurven von McN-A-343 ($10^{-7}$-$2x10^{-6}$M) parallel an jeweils 8 Organabschnitten erstellt (Kontrollkurven). Nach 3 Kontrollkurven wurde eine feste Konzentration des Agonisten (z.B. $5x10^{-7}$, $10^{-6}$, $3x10^{-6}$, $10^{-5}$ bzw. $3x10^{-5}$M) pro Organabschnitt appliziert und nach Stabilisierung des Effektes steigende Konzentrationen des zu prüfenden Antagonisten hinzugefügt. Die verwendete Nährlösung hatte folgende Zusammensetzung (mM): NaCl 118, KCl 4,7, $CaCl_2x2H_2O$ 2,5, $MgSO_4x7H_2O$ 0,6, $KH_2PO_4$ 1,2, $NaHCO_3$ 25,0 und Glukose 11,1. Zur Blockade von $\alpha_2$-Adrenozeptoren enthielt die Nährlösung zusätzlich $10^{-6}$M Yohimbine.

Methode 2: Elektrisch gereizter linker Vorhof der Ratte (Eltze et al., 1985, Europ. J. Pharmacol. 112, 211-224).

Registriert wurden isometrische Kontraktionen (HSE-Kraftaufnehmer, K-30; Watanabe Linear Corder Mark 5) isolierter, linker Vorhöfe von Ratten (männlich, 250-350 g) im Organbad (10 ml, Tyrode-Nährlösung, 31°C, Begasung mit Carbogen, Vorspannung der Organe 0,25 g) unter elektrischer Stimulation (HSE-Reizgerät; hakenförmige Pt-Elektrode am Badgrund und eingetauchte, ringförmige Pt-Elektrode 1 cm unter der Wasseroberfläche des Bades; 7 V, 3 ms, 2 Hz). Nach einer Äquilibrierungszeit von 30 Minuten wurden nach der von van Rossum (1963) beschriebenen Technik kumulative Dosiswirkungskurven von Carbachol ($10^{-8}$-$10^{-6}$ M, in Gegenwart des Antagonisten auch bis $10^{-5}$ oder $10^{-4}$ M) parallel an jeweils 4 Organen erstellt. Die zu prüfende Substanz wurde 20 Minuten vor der Carbachol-Applikation den Organbädern zugefügt. Die Pausen zwischen den einzelnen Dosiswirkungskurven betrugen 30 - 45 Minuten.

Die verwendete Tyrode-Nährlösung hatte folgende Zusammensetzung (mM): NaCl 130, KCl 2,0, $CaCl_2x2H_2O$ 1,8, $MgCl_2x6H_2O$ 0,98, $NaHCO_3$ 12,0, $NaH_2PO_4$ 0,42 und Glukosemonohydrat 5,6.

$pA_2$-Werte (-log der molaren Dissoziationskonstanten des kompetitiven Antagonist-Rezeptor Komplexes) wurden im Schild-Plot durch lineare Regression ermittelt: $pA_2 = -\log [B/(x-1)]$ , (Arunlakshana und Schild, 1959).

In der nachfolgenden Tabelle 7 sind $pA_2$-Werte der Substanzen angegeben, bei denen die Steigung der Regressionsgeraden für theoretisch kompetitiven Antagonismus auf 1,00 normiert wurden (Tallarida et al., 1979).

Tabelle 7

Affinität von (±)-4,9-Dihydro-3-methyl-4-[(4-methyl-1-piperazinyl)-acetyl]-10H-thieno[3,4-b][1,5]benzodiazepin-10-on (Telenzepin, = Verbindung 2) und seinen Konformeren [(+)-Enatiomeres = Verbindung 1, (-)-Enantiomeres = Verbindung 3] zu $M_1$- und $M_2$-Rezeptoren

| Substanz | $M_1$-Rezeptor (ganglionär) Vas deferens des Kaninchens | | $M_2$-Rezeptor (cardial) Linker Vorhof der Ratte | |
|---|---|---|---|---|
| | $pA_2 \pm$ SEM | n | $pA_2 \pm$ SEM | n |
| Verbindung 1 | 9,12 ± 0,08 | 7 - 18 | 7.67 ± 0,08 | 15 - 20 |
| Verbindung 2 | 8.86 ± 0,06 | 20 | 7,32 ± 0,09 | 6 - 11 |
| Verbindung 3 | 6,98 ± 0,14 | 11 - 18 | 6.12 ± 0,06 | 11 - 15 |

Ref.: Van Rossum J. M., 1963, Arch. int. Pharmacodyn. 143, 299. Eltze, M. et al., 1985, Europ. J. Pharmacol. 112, 211. Arunlakshana O. and H.O. Schild, 1959, Br. J. Pharmacol. 14, 48. Tallarida R. J. et al., 1979, Life Sci. 25, 637. Eltze M., 1988, Submitted for publication in Europ. J. Pharmacol.

Prüfung der antiulcerogenen und sekretionshemmenden Wirkung an der modifizierten Shay-Ratte

Die ausgezeichnete Magenschutzwirkung und die magensekretionshemmende Wirkung der Verbindung (+)-4,9-Dihydro-3-methyl-4-[(4-methyl-1-piperazinyl)acetyl]-10H-thieno[3,4-b][1,5]benzodiazepin-10-on (in der folgenden Tabelle 8 = Verbindung 1) im Vergleich zu dem korrespondierenden (-)-Enantiomeren (= Verbindung 3) und zu dem Racemat (= Verbindung 2) kann in Untersuchungen an der modifizierten Shay-Ratte nachgewiesen werden. In der folgenden Tabelle 8 ist der Einfluß der genannten Verbindungen nach intravenöser (i. v.) Gabe auf die Läsionsbildung sowie die Säureausscheidung bei der modifizierten Shay-Ratte dargestellt.

Tabelle 8

| Lfd. Nr. | N (Anzahl der Tiere) | Magenschutzwirkung Minderung des Läsionsindexes, ca. ED50+) (mg/kg) i.v. | Säuresekretionshemmung Verringerung der HCl-Sekretion, ca. ED50+) (mg/kg) i.v. |
|---|---|---|---|
| 1 | 24 | 0,01 | 0,085 |
| 2 | 24 | 0,042 | 0,18 |
| 3 | 24 | 0,65 | 3,0 * |

+) ED50 = Dosis (interpoliert), die den Läsionsindex bzw. die HCl-Sekretion des Rattenmagens bei der behandelten Gruppe gegenüber der Kontrollgruppe um 50 % mindert.
* Hemmung nach 3 mg: 46 %

Methodik

Die Ulcusprovokation erfolgt an 24 Stunden nüchternen Ratten (weiblich, 180-200 g, 4 Tiere je Käfig auf hohem Gitterrost) durch Pylorusligatur (unter Diethylethernarkose) und orale Applikation von 100 mg/10ml/kg Acetylsalicylsäure. Die zu prüfenden Substanzen werden intravenös (1 ml/kg) unmittelbar nach der Pylorusligatur verabreicht. Der Wundverschluß wird mittels Michelklammern vorgenommen. 4 Stunden danach erfolgt die Tötung der Tiere im Etherrausch durch Atlas-Dislokation und die Resektion des Magens.

Der Magen wird längs der großen Kurvatur eröffnet und auf einer Korkplatte aufgespannt, nachdem zuvor die Menge des sezernierten Magensaftes (Volumen) und später sein HCl-Gehalt (Titration mit Natronlauge) bestimmt wird. Mit einem Stereomikroskop werden bei 10-facher Vergrößerung Anzahl und Größe (=Durchmesser) vorhandener Ulcera ermittelt. Das Produkt aus Schweregrad (gemäß nachfolgender Punkteskala) und Anzahl der Ulcera dient als individueller Läsionsindex.

| Punkteskala: | | |
|---|---|---|
| keine Ulcera | | 0 |
| Ulcusdurchmesser | 0,1 - 1,4 mm | 1 |
| | 1,5 - 2,4 mm | 2 |
| | 2,5 - 3,4 mm | 3 |
| | 3,5 - 4,4 mm | 4 |
| | 4,5 - 5,4 mm | 5 |
| | > 5,5 mm | 6 |

Als Maß für den antiulcerogenen Effekt dient die Minderung des mittleren Läsionsindex jeder behandelten Gruppe gegenüber dem der Kontrollgruppe (=100 %). Die ED50 bezeichnet diejenige Dosis, die den mittleren Läsionsindex bzw. die HCl-Sekretion gegenüber der Kontrolle um 50 % mindert.

**Patentansprüche**

1. Verwendung des 4,9-Dihydro-3-methyl-4-[(4-methyl-1-piperazinyl)acetyl]-10H-thieno[3,4-b][1,5]benzodiazepin-10-ons und seiner pharmakologisch verträglichen Salze zur Herstellung von Arzneimitteln zur Behandlung und Prophylaxe von Erkrankungen der Bronchien.

2. Verwendung des (+)-4,9-Dihydro-3-methyl-4-[(4-methyl-1-piperazinyl)acetyl]-10H-thieno[3,4-b][1,5]benzodiazepin-10-ons und seiner pharmakologisch verträglichen Salze zur Herstellung von Arzneimitteln zur Behandlung und Prophylaxe von Erkrankungen der Bronchien.

3. Verfahren zur Herstellung des (+)- und (-)-Konformeren des 4,9-Dihydro-3-methyl-4-[(4-methyl-1-piperazinyl)acetyl]-10H-thieno[3,4-b][1,5]benzodiazepin-10-ons (= Verbindung der Formel I), dadurch gekennzeichnet, daß man das Konformerengemisch der Verbindung der Formel I

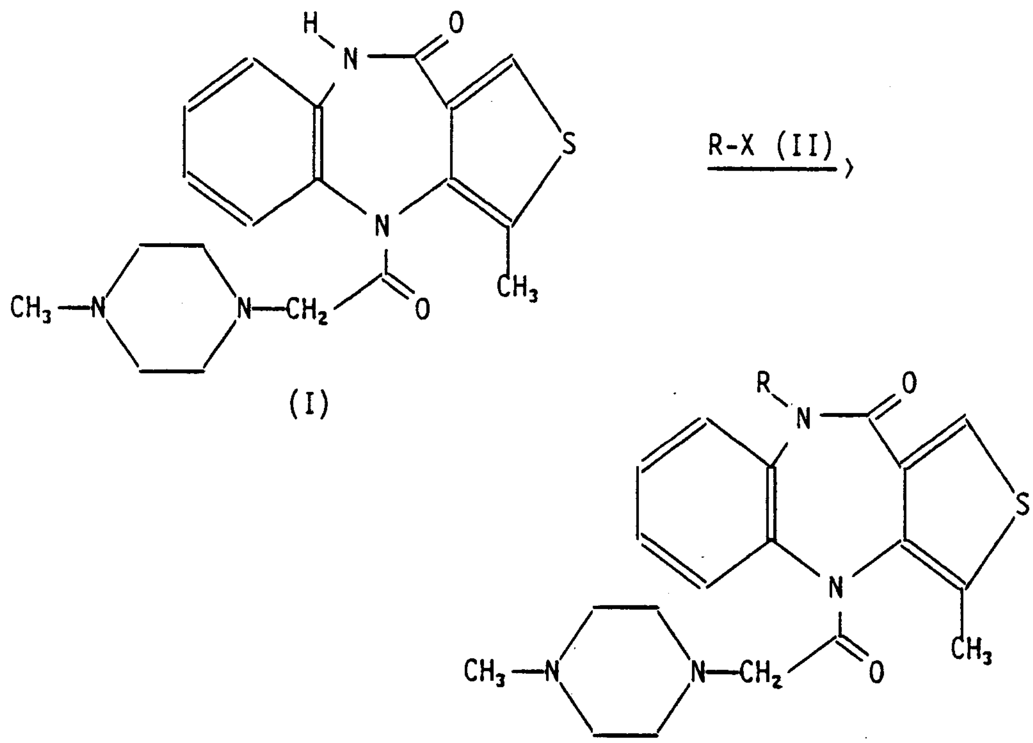


(I)

(III)

mit einer konfigurativ einheitlichen chiralen Verbindung der Formel II, worin R einen konfigurativ einheitlichen, chiralen Rest und X eine Abgangsgruppe darstellt, umsetzt, das erhaltene Diastereomerengemisch III trennt und aus den optisch reinen Diastereomeren die konformativ einheitlichen Verbindungen I freisetzt.

4. Verfahren nach Anspruch 3, dadurch gekennzeichnet, daß R einen Isopinocampheyloxymethylrest darstellt.

5. (+)-4,9-Dihydro-3-methyl-4-[(4-methyl-1-piperazinyl)-acetyl]-10H-thieno-[3,4-b][1,5]benzodiazepin-10-on und seine Salze.

6. Zwischenprodukte der Formel III nach Anspruch 3, worin R die in Anspruch 3 angegebene Bedeutung hat, und ihre Salze.

7. Zwischenprodukte der Formel III nach Anspruch 3, worin R die in Anspruch 4 angegebene Bedeutung hat, und ihre Salze.

8. Arzneimittel enthaltend (+)-4,9-Dihydro-3-methyl-4-[(4-methyl-1-piperazinyl)-acetyl]-10H-thieno-[3,4-b][1,5]benzodiazepin-10-on und/oder seine pharmakologisch verträglichen Salze.

## Claims

1. Use of 4,9-dihydro-3-methyl-4-[(4-methyl-1-piperazinyl)-acetyl]-10H-thieno[3,4-b][1,5]benzodiazepin-10-one and its pharmacologically tolerated salts for the preparation of medicaments for the treatment and prophylaxis of diseases of the bronchi.

2. Use of (+)-4,9-dihydro-3-methyl-4-[(4-methyl-1-piperazinyl)-acetyl]-10H-thieno[3,4-b][1,5]benzodiazepin-10-one and its pharmacologically tolerated salts for the preparation of medicaments for the treatment and prophylaxis of diseases of the bronchi.

3. A process for the preparation of the (+)- and (-)-conformer of the 4,9-dihydro-3-methyl-4-[(4-methyl-1-piperazinyl)-acetyl]-10H-thieno[3,4-b][1,5]benzodiazepin-10-one (= compound of the formula I), characterised in that the conformer mixture of the compound of the formula I

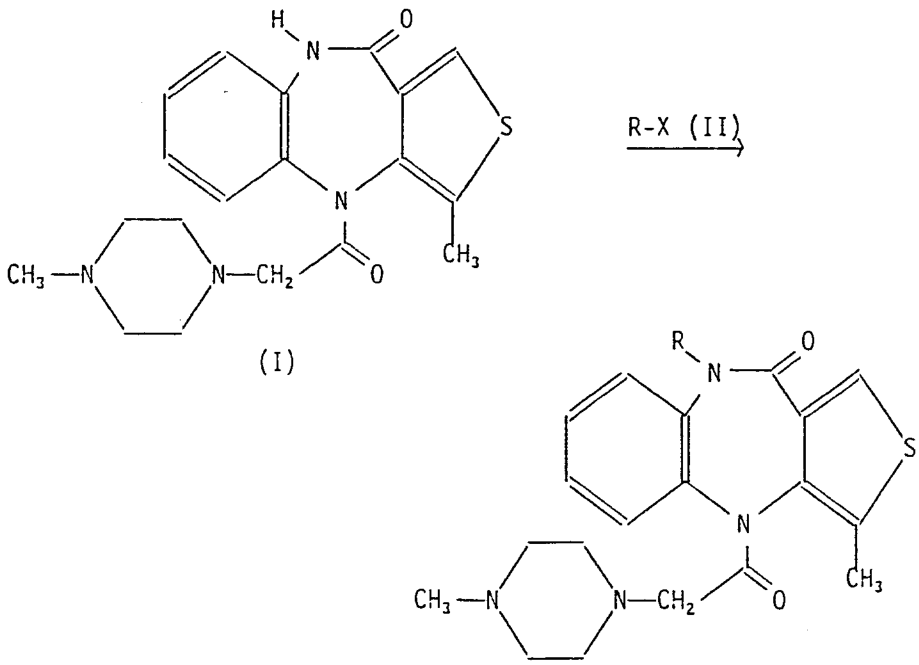


(I)

(III)

is reacted with a chiral compound which has a uniform configuration and is of the formula II, wherein R represents a chiral radical having a uniform configuration and X represents a leaving group, the resulting diastereomer mixture III is separated and the compounds I having a uniform conformation are liberated from the optically pure diastereomers.

4. Process according to Claim 3, characterised in that R represents an isopinocampheyloxymethyl radical.

5. (+)-4,9-dihydro-3-methyl-4-[(4-methyl-1-piperazinyl)-acetyl]-10H-thieno[3,4-b][1,5]benzodiazepin-10-one and its salts.

6. Intermediates of the formula III according to Claim 3, wherein R has the meaning stated in Claim 3, and its salts.

7. Intermediates of the formula III according to Claim 3, wherein R has the meaning stated in Claim 4, and its salts.

8. Medicaments containing (+)-4,9-dihydro-3-methyl-4-[(4-methyl-1-piperazinyl)-acetyl]-10H-thieno[3,4-b][1,5]benzodiazepin-10-one and/or its pharmacologically compatible salts.

## Revendications

1. Utilisation du 4,9-dihydro-3-méthyl-4-[(4-méthyl-1-pipérazinyl)-acétyl]-10H-thiéno[3,4b][1,5]-benzodiazépin-10-one et de ses sels tolérés sur un plan pharmacologique pour la fabrication de médicaments destinés au traitement et à la prophylaxie des affections des bronches.

2. Utilisation du (+)-4,9-dihydro-3-méthyl-4-[(4-méthyl-1-pipérazinyl)-acétyl]-10H-thiéno[3,4b][1,5]-benzodiazépin-10-one et de ses sels tolérés sur un plan pharmacologique pour la fabrication de médicaments destinés au traitement et à la prophylaxie des affections des bronches.

3. Procédé de fabrication des conformères (+) et (-) du 4,9-dihydro-3-méthyl-4-[(4-méthyl-1-pipérazinyl)-acétyl]-10H-thiéno-[3,4-b][1,5]-benzodiazépin-10-one (=composé de la formule I), caractérisé en ce que l'on convertit le mélange de conformères du composé de la formule I

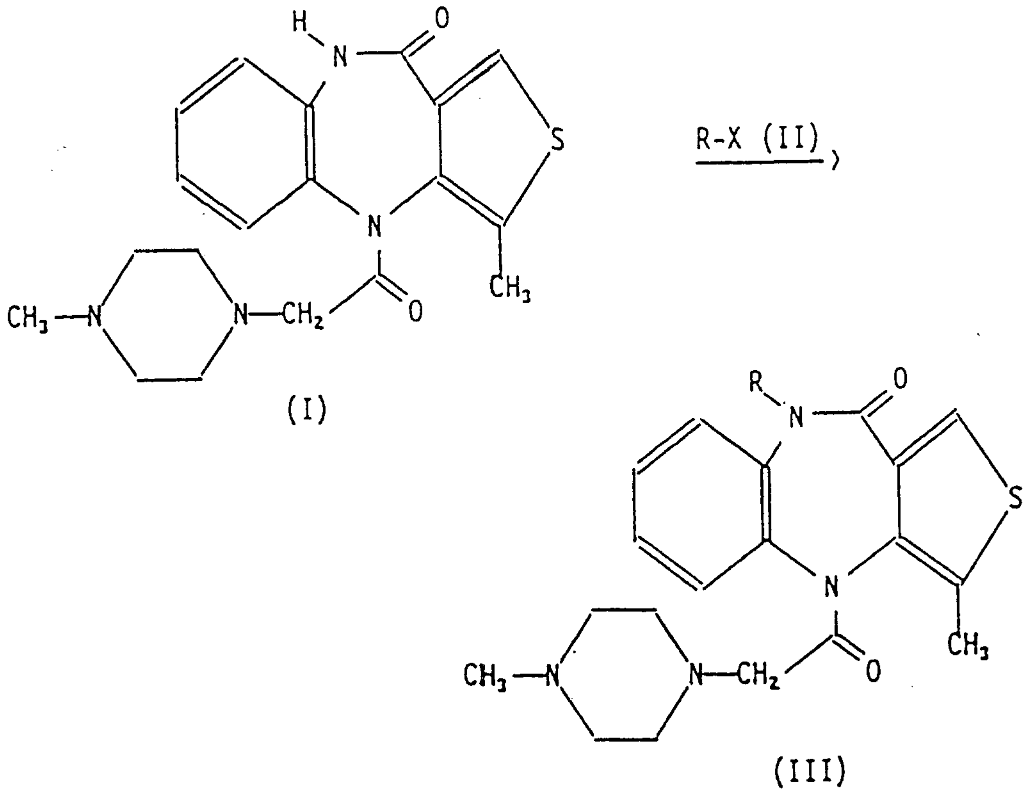


(I)

(III)

avec un composé chiral de la formule II, homogène sur le plan de la configuration, R représentant un résidu chiral homogène sur le plan de la configuration et X un groupe dérivé et que l'on sépare le mélange de diastéréomères III obtenu et qu'on libère les composés I homogènes sur le plan de la conformation à partir des diastéréomères optiquement purs.

4. Procédé selon la revendication 3, caractérisé en ce que R représente un résidu d'isopinocampheyloxyméthyle.

5. (+)-4,9-dihydro-3-méthyl-4-[(4-méthyl-1-pipérazinyl)acétyl]-10H-thiéno[3,4-b][1,5]-benzodiazépin-10-one et ses sels.

6. Produits intermédiaires de la formule III selon la revendication 3, R ayant la signification indiquée dans la revendication 3 et leurs sels.

7. Produits intermédiaires de la formule III selon la revendication 3, R ayant la signification indiquée dans la revendication 4 et leurs sels.

8. Médicament comprenant du (+)-4,9-dihydro-3-méthyl-4-[(4-méthyl-1-pipérazinyl)-acétyl]-10H-thiéno[3,4-b][1,5]benzodiazépin-10-one et/ou ses sels tolérés sur un plan pharmacologique.